# EUROPEAN PATENT APPLICATION

(11) **EP 2 237 039 A1**
(43) Date of publication of application: **06.10.2010**
(21) Application number: 09157342.8
(22) Date of filing: 03.04.2009
(51) Int. Cl.: G01N 33/58, G01N 33/50

(54) **Encoded microparticles**

(71) Applicant: Universitat Autònoma De Barcelona, 08193 Bellaterra (Barcelona) (ES); Consejo Superior De Investigaciones Científicas, 28006 Madrid (ES)
(72) Inventor: Santaló Pedro, Josep, 08193, Bellaterra (Barcelona) (ES); Barrios Sanromà, Lleonard, 08193, Bellaterra (Barcelona) (ES); Ibáñez De Sans, Elena, 08193, Barcelona (ES); Nogués Sanmiguel, Carme, 08193, Bellaterra (Barcelona) (ES); Esteve Tinto, Jaume, 28006, Madrid (ES); Plaza Plaza, José Antonio, 28006, Madrid (ES)
(74) Representative: ZBM Patents

(57) **Abstract**

The present invention relates to an encoded microparticle for labeling an isolated cell or an isolated embryo **characterized in that** it is made of a biocompatible material and its external shape comprises a code by which it can be identified. The use of an encoded microparticle for labeling and/or tracking isolated biological material. A method of tracking an encoded microparticle in or attached to an isolated cell or embryo using an optical microscope, preferably an inverted optical microscope with an objective substantially between 20X - 100X.

## Description

### TECHNICAL FIELD OF THE INVENTION

Microparticles or nanoparticles are generally referred to as structures whose characteristic dimensions are on the order of micrometers or less. According to some definitions, microparticles are particles between 0.1 and 100 µm in size. According to these definitions, nanoparticles are particles 1 and 100 nm in size. Due to their small dimensions, nanoparticles and microparticles have unique properties (e.g. much larger surface-to-volume ratio) that make them behave differently than macroparticles.

Following these definitions, the present invention relates to microparticles, and more particularly to encoded microparticles.

### BACKGROUND OF THE INVENTION

An increasing demand for tracking smaller items has driven the exploration for novel methods of barcoding at much smaller scale. Individual cell tracking is of great interest in cell biology to evaluate individual cell behavior (such as e.g. cell survival, cell movement, relationship with other cells) under different conditions (such as e.g. exposure to toxic gases or compounds, to a source of light (phototaxy), to a chemical stimulus (chemotaxy) or to X-ray microbeams).Therefore, in the last decade, there has been a growing interest in developing different types of barcodes to track living cells either in vivo or in vitro.

Prior art solutions to the problems of tracking individual cells or embryos comprise for example: quantum-dots-tagged microbeads, metallic barcodes, porous-silicon photonic crystals {{Cunin,F. 2002}}, acrylic encoded carriers {{Beske,O. 2004}}, iron-oxide magnetic nanoparticles, PDMS particles {{Dendukuri,D. 2006}}, nanodisk codes {{Qin,L. 2007}} or diamond nanoparticles {{Faklaris,O. 2008}}.

These prior art particles generally suffer from one or more of the following problems: some cannot be traced using relatively simple tools or machinery, such as an optical microscope, but instead they need an electron microscope and/or dedicated software to interpret the images. Other particles need to be coupled to different fluorochromes to act as a barcode, and this extra step makes barcode manufacturing more expensive and difficult. Additionally, the use of fluorochromes requires a fluorescence microscope or a confocal scanning laser microscope to visualize the barcode but UV light and laser beams have been reported to be harmful for living cells {{McCarthy,J.R. 2006}}. Finally, some particles are plainly not suitable for being used to track either cells or embryos, because they are not made of a biocompatible material.

Another area of interest for tracking smaller "items" is in assisted reproduction centers. High numbers of patients in such centres mean that the assisted reproduction process (e.g. in vitro fertilization) cannot be completely separated for each individual. In such centres, it nowadays is frequently not possible to dedicate separate work and storage space for each individual specimen. This has led to problems of mixing up of specimens. Cases have been reported where children were born after in vitro fertilization, that had fenotypes decisively different from their supposed biological parents (e.g. of different race). There thus clearly exists a need for improving traceability of samples or specimens in this line of business.

There thus still exists a need for devices that may be used to label and track isolated (living) cells or embryos. The object of the present invention fulfills this need. The object is achieved by an encoded microparticle according to claim 1, a use of such a microparticle according to claim 14 and a method of tracking such a microparticle according to claim 20.

### SUMMARY OF THE INVENTION

In a first aspect, the present invention provides an encoded microparticle for labeling an isolated cell or an isolated embryo **characterized in that** it is made of a biocompatible material and its external shape comprises a code by which it can be identified.

According to the present invention, the encoded microparticle can be used for labeling or tracking isolated cells (e.g. oocytes, or zygotes) or embryos. Its dimensions are small enough that it can be introduced into or attached to said isolated cells or embryos. Contrary to many prior art labeling and tracking devices, the code of the microparticle is comprised in its external shape. The code comprised in the particle may thus be considered a spatial code. There is no need for e.g. fluorochromes to be able to identify the code. This, and the fact that the microparticle is made from a biocompatible material makes its application in isolated (living) cells or embryos possible.

Preferably, the dimensions of the microparticles are such that its code can be distinguished using an optical microscope and such that it can be introduced into or attached to a cell without affecting the viability of a cell. If the dimensions are sufficiently large for the code to be viewed under an optical microscope, the identification and tracking is considerably facilitated as compared to e.g. the use of an electron microscope. Simultaneously, the dimensions of the microparticle should be such that it does not influence the viability of the cell or embryo.

In some embodiments of the invention, the encoded microparticle is made of polysilicon or silicon. In some embodiments, said polysilicon or silicon material is provided with an outer layer of a lectin, preferably wheat germ agglutinin. Other suitable materials for the microparticle may be any other biocompatible material that allows its manufacture at microsize. Polysilicon and silicon have been proven to be biocompatible and not affect the viability of the cells into which they are introduced or to which they are attached. It has been found that microparticles made of polysilicon or silicon covered with a layer of wheat germ agglutinin are especially suitable for being attached to the zona pellucida of an embryo in vitro.

In preferred embodiments of the invention, the external shape of the microparticle comprises a distinctive marker allowing determination of the microparticle's orientation. In this way, the external shape of the particle (i.e. its code) can only be interpreted in a single way.

In preferred embodiments, the external shape comprises a base element and a plurality of optional segments and gaps, said optional segments and gaps defining the code by which the microparticle can be identified. Each optional segment or gap can be considered as a bit, which can have a value of 1 (segment) or gap (0). The shape of the microparticle is thus represented by a binary code e.g. 1111 1111 (corresponding to number 255) or e.g. 1101 1000 (corresponding to number 216). To further increase the number of possible identifiers within a single microparticle, the length of such optional segments may also be varied.

In some embodiments, its base element is a cylinder and said plurality of optional segments is a plurality of annular protrusions around the circumference of the cylinder. In other embodiments, its base structure is substantially flat and substantially rectangular and said plurality of optional segments is a plurality of substantially rectangular segments within said base structure. In yet other embodiments, its base structure is substantially flat and comprises at least a substantially rectangular part, and said plurality of optional segments is a plurality of polygonal segments arranged around the perimeter of said rectangular part. Within the scope of the present invention, any external shape which allows unique identification may be used.

In some embodiments, the dimensions of the optional segments and gaps are approximately 1 µm or more. Such dimensions allow relatively easy identification using an optical microscope. In some embodiments, substantially flat and rectangular base structures provided with optional segments and gaps are used. In these embodiments, suitable length and width are approximately between 2-25 µm and between 3 - 15 µm respectively, preferably between 6-14 µm and between 4-8 µm respectively. The upper limit of the length and width is set so as not to affect the viability of the cells. The lower limit may be determined by the user: if a larger number of bits (optional segments and gaps) is desired, length and width should be increased, if instead a lower number of bits is needed, length and width may be reduced. In these embodiments, the thickness of the base structure is preferably between approximately 0.3 µm - 0.8 µm, more preferably approximately 0.5 µm. It is generally desirable to reduce the thickness of the microparticle as much as possible. The thickness of flat microparticles namely does not influence on the identificability of the particles (the ability to identify the codes of the particles is mainly determined by the microparticle's length and width, particularly the length and width of the optional segments). By reducing the thickness the occupied volume in a cell may be reduced, thus increasing its viability. However, by reducing the thickness too much, the microparticles may more easily break, which is clearly not desirable.

According to another aspect of the current invention, one or a plurality of encoded microparticles may be used for labeling and/or tracking isolated biological material. As previously explained, the microparticles according to the present invention are especially suitable for this kind of application. Experiments that have been carried out have shown the use and success of labeling and/or tracking a living isolated cell, particularly a human macrophage.

Other experiments have shown the capability of labeling and/or tracking a zygote or embryo in vitro without altering its development potential. This thus proves the use of the invention in assisted reproduction centres. It has been shown by inventors that labeling and/or tracking is possible by introducing at least one encoded microparticle in the perivitelline space or by attaching at least one encoded microparticle to the zona pellucida.

For labeling and/or tracking an isolated cell or embryo, at least one encoded microparticle may be used. A plurality of encoded microparticles may be used to simplify the labeling and tracking (it will be easier to read the code of at least one of the microparticles). However, the increased number of encoded microparticles should not affect the viability of the biological material. The maximum number of microparticles used for labeling and/or tracking a single isolated cell or isolated embryo may therefore depend on various factors: size of microparticles, size of cell, type of cell etc.

In yet another aspect, the present invention relates to a method of tracking an encoded microparticle introduced in or attached to an isolated cell, or embryo using an optical microscope, preferably an inverted optical microscope with an objective substantially between 20X - 100X.

### BRIEF DESCRIPTION OF THE DRAWINGS

Particular embodiments of the present invention will be described in the following, only by way of non-limiting example, with reference to the appended drawings, in which:
Figures 1 (a)-(c) shows a first embodiment of a shape of an encoded microparticle according to the present invention;
Figures 2 (a) and (b) illustrate the number of possible shapes (the number of possible codes) which may be assumed by a encoded microparticle such as shown in figure 1;
Figures 3 (a)-(f) illustrate a process by which microparticles according to the present invention may be manufactured;
Figures 4 (a) and (b) show scanning electron microscope images of different encoded microparticles according to the present invention;
Figure 5 shows an image taken with an inverted optical microscope with a 40X objective of an in vitro culture of macrophage cells with polysilicon encoded microparticles according to the present invention;
Figures 6 (a) and (b) show images taken with an inverted optical microscope of two macrophages with an encoded microparticle according to the present invention in their cytoplasm;
Figure 7 shows a result of experiments using macrophages comprising encoded polysilicon microparticles according to the present invention and control macrophages, illustrating the viability of macrophages comprising the encoded microparticles;
Figure 8 shows daily movements during 10 days of a 8 different macrophages in culture with an encoded microparticle according to the present invention in their cytoplasm;
Figure 9 shows an image taken with an inverted optical microscope illustrating the movement of a macrophage comprising an encoded microparticle;
Figures 10 (a)-(c) illustrate encoded microparticles according to the present invention of three different types;
Figure 11 illustrates the development of embryos comprising a single encoded microparticle in their perivitelline space;
Figure 12 (A)-(G) illustrates various embryos at various stages comprising encoded microparticles of various types in its perivitelline space;
Figures 13 (A) and (B) show two different views of the same embryo comprising four encoded microparticles of the type shown in figure 10(c);
Figure 14 shows results of experiments that illustrate the rate at which embryos could be identified according to the number of microparticles injected;
Figures 15 (A1)-(C5) show the embryos comprising encoded microparticles according to the present invention at various stages of embryonic development;
Figures 16(A1)-(B2) show the liberation of an encoded microparticle according to the present invention at the moment of hatching of the blastocyst;
Figures 17 and 18 show two images taken with a scanning electron microscope of a plurality of encoded particles of the type shown in figure 10 (a);
Figure 19 shows an image taken with an inverted optical microscope of a mouse embryo comprising a plurality of polysilicon microparticles comprising a layer of lectin attached to the zona pellucida;

### DETAILED DESCRIPTION

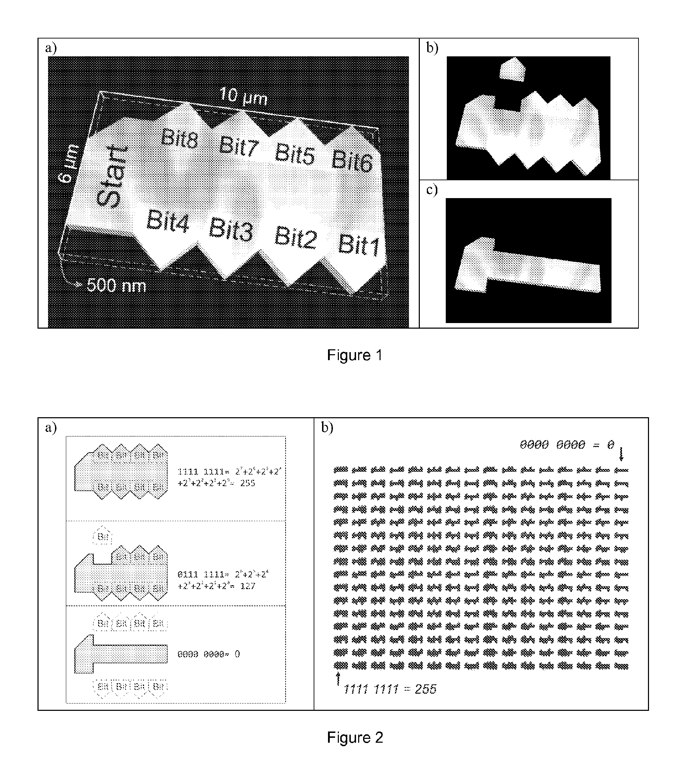

Figures 1 (a)-(c) show a first embodiment of a shape of an encoded microparticle according to the present invention. In the shown embodiment, the microparticle comprises a base structure which is substantially flat and which comprises at least a substantially rectangular part, and a plurality of optional polygonal segments arranged around the perimeter of said rectangular part. Each optional segment (in the shown embodiment a hexagon, but within the scope of the present invention, this shape may be varied) represents a 1, each optional gap represents a 0. The external shape of the microparticle forms a code comprising a total of 8 bits (1 or 0).

The encoded microparticle is asymmetrical and comprises a marker (in the upper left corner of the particle shown in figure 1a) which allows the determination of the particle's orientation. This way, the code represented by the shape of the microparticle can only be interpreted in a single way.

The device shown in figure 1 has a length of approximately 10 µm and a width of approximately 6 µm, and a thickness of approximately 0.5 µm. Within the scope of the present invention, these dimensions may be varied. In the most preferred embodiments of the invention the shape of the microparticle can be identified using an optical microscope. A suitable resolution using this kind of microscope is approximately 1 µm. In preferred embodiments, the dimensions of the optional segments (the bits) thus should be approximately 1 µm or more. It is furthermore important, that when introduced into a cell or an embryo, the microparticle does not influence the viability of such cell or embryo. This determines the maximum possible dimensions of the microparticles according to the present invention.

The microparticle's dimensions were chosen to fulfill requirements in terms of cell viability and identificability. The devices are small enough to be introduced into different cell types. Typical cells have volumes in the range from 500 to 14,000 µm³. In addition, as most cell studies are performed using light and confocal microscopes, features smaller than 1 µm could be difficult to identify. Thus, considering the code visibility, the lateral dimensions of the encoded microparticles were fixed to 10 µm x 6 µm.

In contrast, the thickness of the microparticle was fixed to submicron dimensions, 500 nm, according to the small volume criteria. Due to the submicron range of the thickness of the microparticle, the maximum volume of an individual microparticle was approximately 30 µm³; thus the volume of the particle of this embodiment is approximately 20 to 500 times smaller than that of many typical cells.

Figures 2 (a) and (b) illustrate the number of possible shapes (the number of possible codes) which may be assumed by an encoded microparticle such as shown in figure 1. The data capacity, i.e. the number of possible different codes, depends on the number of bits of the encoded microparticle. The design shown in figures 1 and 2 has 8 bits, which means 256 different codes, from 0 to 255. Figure 2a shows three different codes. If all the pentagonal segments are present, the binary code is 1111 1111 (which in decimal representation corresponds to the number 255); if the pentagon Bit8 is 0 (there is a gap at that location), the binary code is 0111 1111 that in decimal corresponds to the number 127; and if no pentagonal segments are formed on the perimeter of the base structure, the binary code is 0000 0000 (which corresponds to the number 0). A schematic view of the 256 different codes is shown in Figure 2b.

Within the scope of the present invention, the number of optional segments and gaps (i.e. the number of bits may be varied). If e.g. the length of the microparticle with the same shape is increased to 14 µm, the number of bits may be increased to e.g. 12 (assuming a width of each optional segment between 1 and 2 µm). A microparticle of this length, with the same shape as before, thus allows 2¹² = 4096 possible codes. This number can easily be further increased by allowing e.g. variable length of the optional segments.

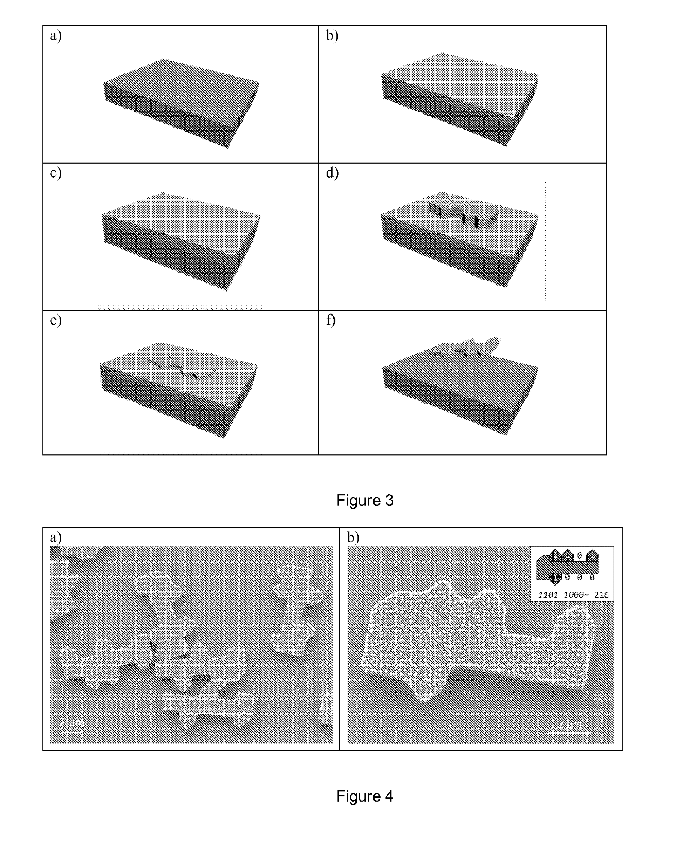

Figures 4 (a) and (b) show scanning electron microscope images of different encoded microparticles of the same type as shown in figures 1 and 2.

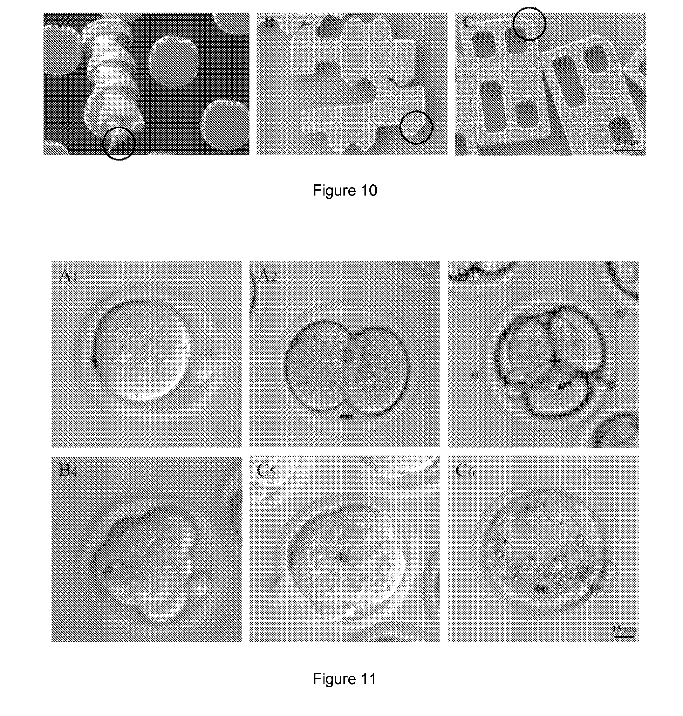

Figures 10 (A)-(C) illustrate encoded microparticles according to the present invention of three different types. The type shown in figure 10 (B) (from hereinafter called type B) corresponds to the ones shown in figures 1, 2 and 4, comprising a plurality of optional polygonal segments arranged around the perimeter of a rectangular part of a base structure.

Figure 10 (A) shows another type of encoded microparticles according to the present invention (from hereinafter called type A), which comprises a cylindrical base structure. Around said cylindrical base structure, a number of optional annular protrusions are provided, each annular protrusion representing a 1, each gap (the absence of such a protrusion) representing a 0. Another way to regard this same type of structure is that its base element is a cylinder and said plurality of optional gaps is a plurality of annular retrusions on the circumference of the cylinder.

Figure 10 (C) shows yet another type of encoded microparticle according to the present invention (from hereinafter called type C). This microparticle comprises a base structure which is substantially flat and substantially rectangular and a plurality of substantially rectangular optional segments within said base structure. Every segment represents a 1, and every hole represents a 0.

All encoded microparticles shown in figure 10 are asymmetrical, which allows the determination of their orientation. The unique marker which allows the determination of said orientation univocally, is indicated within the black circles of figures 10 (A)-(C). The marker in figure 10 (A) is the thin, pointy end of the "screw-shaped" microparticle (due to the chosen manufacturing processes, one end of the microparticle is thinner, whereas the other end of the microparticle is wider).

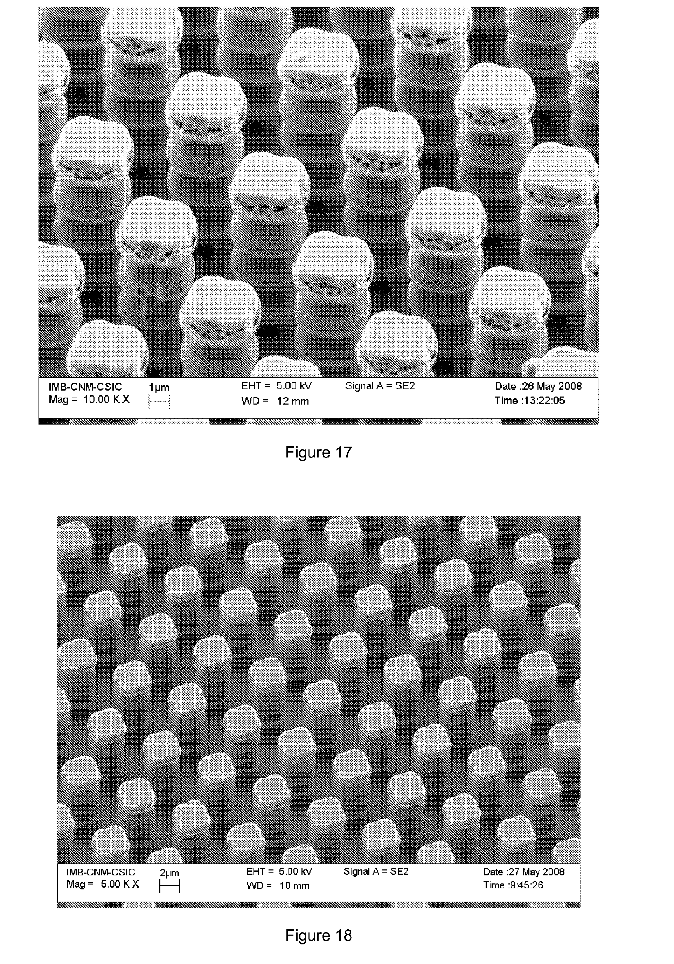

Figures 17 and 18 show two images taken with the scanning electron microscope of a plurality of encoded particles of type A. The scale is indicated in the bottom of the two figures. The plurality of optional annular protrusions (or retrusions) can clearly be distinguished.

As mentioned before, one of the goals of the present invention is to provide an encoded microparticle which can be used for labeling and tracking isolated biological material, such as isolated (living) cells. Experiments were carried out to check whether the encoded microparticles could be introduced succesfully in a cell. Microparticle cellular uptake was analyzed on human macrophages differentiated in vitro and kept in culture for several days.

### The data concerning the experiments:

Macrophage culture: Human monocytic cell line THP-1 (ECACC No.88081201) was maintained in RPMI 1640 medium supplemented with 20% foetal bovine serum (FBS) in standard conditions. THP-1 cells (20,000 cells/well) were plated in 24-well dishes on reticulated glass coverslips in the presence of 0.16µM phorbol 12-myristate 13-acetate (PMA, Sigma) during 3 days to promote macrophage differentiation. Polysilicon microparticles were added in the third day at a rate of 0.5 microparticles/well, adjusted to achieve a maximum number of cells with only one phagocyted microparticle.

Imaging of the microparticles: after 24 hours in contact with polysilicon particles, cell culture medium was changed to remove the non-phagocyted devices and to assure that only microparticles inside cells or in close contact with the cells were left. Particles were localized and identified under an optical inverted microscope (Olympus IX71, Hamburg, Germany) with differential interference contrast. Only macrophages with one phagocyted encoded microparticle were selected and individually followed during 10 days. Images were taken every 24 hours and cell location was recorded. Reticulated coverslips were used to trace individual cell trajectories. Surviving macrophages at day 10 were fixed in Karnovsky's solution (2% paraformaldehyde and 2.5% glutaraldehyde) at room temperature, dehydrated in ethanol series, critical point-dried using CO2 (K850 critical point drier Emitech), mounted on the specimen holder, coated with gold and observed in an S-570 SEM (Hitachi. Tokyo, Japan). Finally, a combined Strata 235 Dual Beam Focused Ion Beam (FIB) and SEM work station (FEI. Hillsboro, OR, USA), was used to section cells to verify that encoded microparticles were inside macrophages.

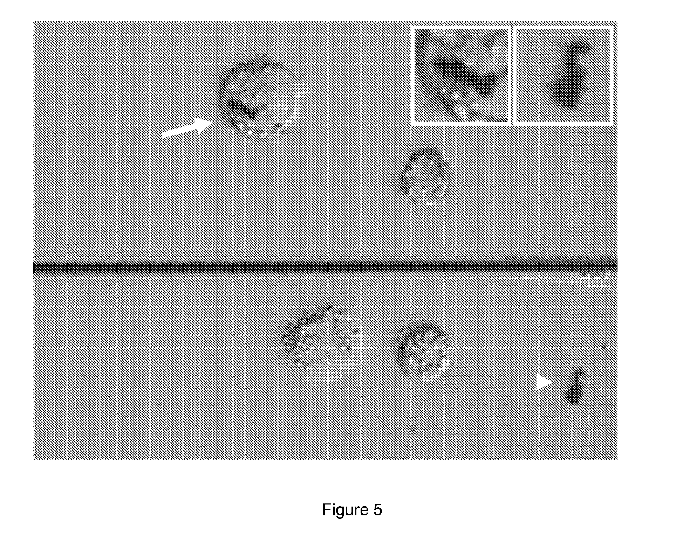

Microparticle cellular uptake was analyzed on human macrophages differentiated in vitro and kept in culture for several days. Macrophages were chosen because they have the capacity to phagocyte large pathogens and it has been described that they can engulf polystyrene particles, at least up to 15 µm {{Foged,C. 2005}}. Once differentiated, macrophages attach to culture plates and can measure up to 90 µm long by 20 µm wide and 15 µm high in the widest region. Macrophage cultures were analyzed 24 hours after encoded microparticle addition to check their cellular uptake. Using an inverted optical microscope, microparticles were localized either inside macrophages (see figure 5) or on their surroundings. To ascertain that the encoded microparticles were inside the cells, and not over or under the plasma membrane, cells were further analyzed with a scanning electron microscope equipped with a Focused Ion Beam work station (SEM-FIB), which allows cutting the cells with extreme accuracy. Several cells which apparently contained an encoded microparticle in their cytoplasm were cut with the FIB to verify this hypothesis. Results confirmed that in all the cells analyzed the encoded microparticle was present inside their cytoplasm (data not shown), thus demonstrating that macrophages are able to phagocyte polysilicon encoded microparticles of 10 µm x 6 µm x 0.5 µm dimensions.

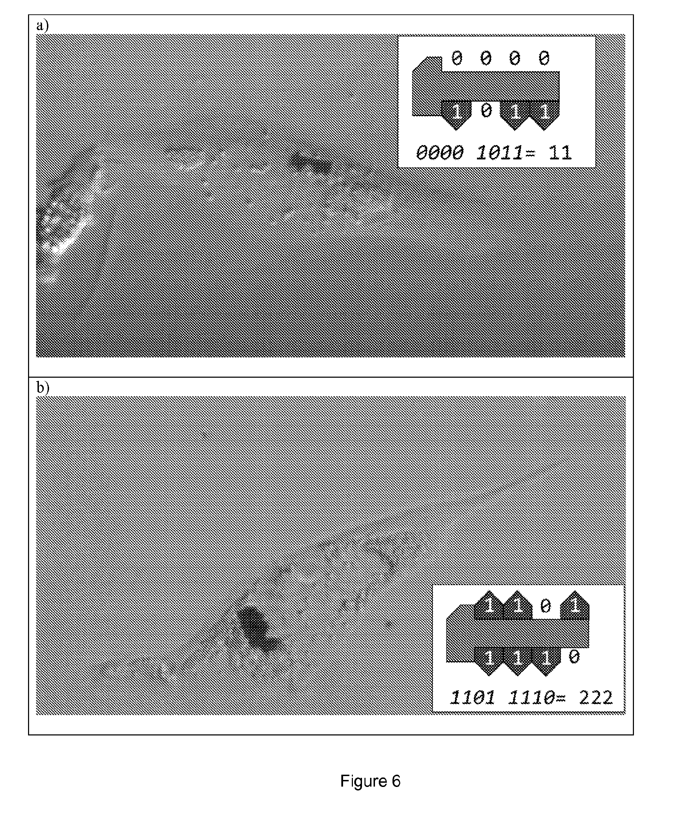

To evaluate the ability to identify the microparticles under an inverted optical microscope, several cells incubated in the presence of various encoded microparticles were selected and photographed with a digital camera. Each individual encoded microparticle could be clearly read using light microscopy. As the microparticles have been designed with a marker that allows determination of its orientation, no interpretation mistakes occurred. In Figure 6, two different encoded microparticles found inside two different macrophages are shown; images were taken using the 60X objective of an inverted microscope (Olympus IX71). The first cell has the binary code 0000 1011 (number 11 in decimal representation) and the second has the binary code 1101 1110 (number 222 in decimal representation). The flat shape of cells facilitates the flat-placed position of the microparticles, allowing their clear identification. Only in very few cases the encoded microparticles were positioned with their base structure in a 90° inclined plane with respect to the focal plane and could not be read.

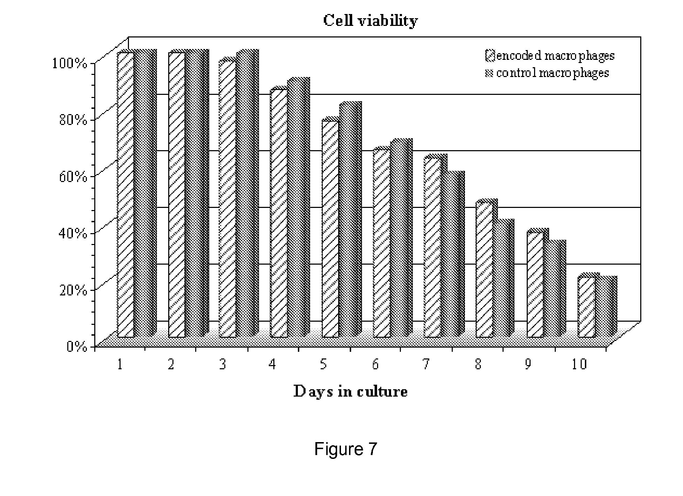

Another important biological requirement is the biocompatibility of the particles. Both biocompatibility and cell viability were tested in further experiments. Macrophages carrying a polysilicon encoded microparticle were studied during 10 days to evaluate cell survival. The initial macrophage population (38 cells) that was studied was reduced down to 20.0% (8 cells) after 10 days in culture (see figure 7); this decrease is comparable with that observed in control cultures (without microparticles; n= 70 cells) of the same macrophage cell line obtained in previous studies carried out in our laboratory (21.1%; also see figure 7). In fact, when survival rates of encoded and control macrophages were compared, no statistically significant differences were found neither in the first five days in culture, nor in the last five days (difference between proportions test). Hence, these results demonstrate that the reduction in macrophage viability can be attributed to the normal behaviour of these cells after differentiation, which attach to the substrate, stop proliferating and finally die {{Tsuchiya,S. 1982}}, and not to the presence of the particles.

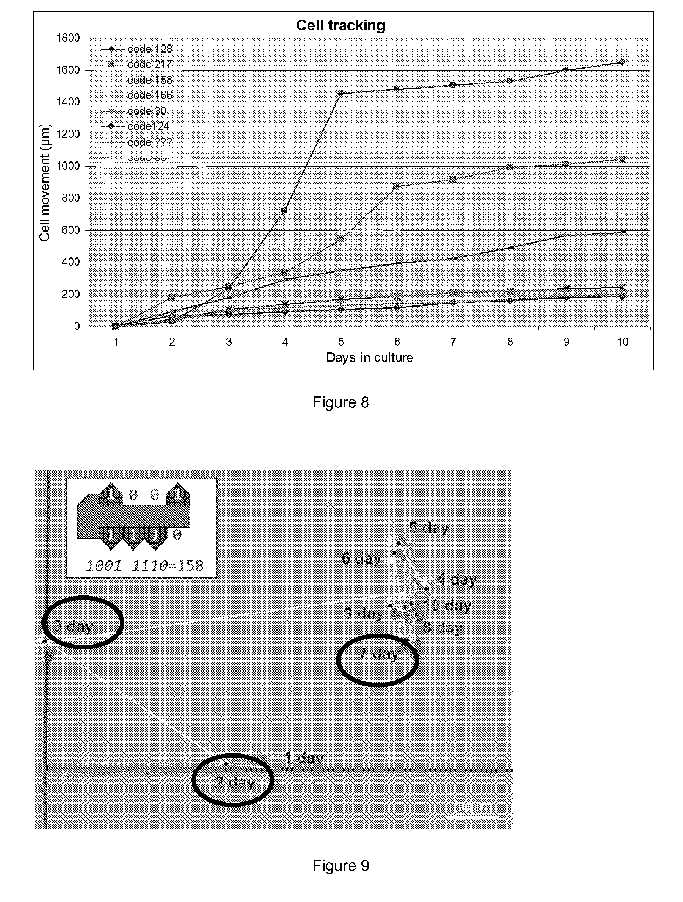

Cell tracking was carried out in 38 different macrophages carrying a polysilicon microparticle. Macrophages were cultured on reticulated coverslips to record the coordinates of each cell containing an encoded microparticle. We were able to localize and identify a particular cell every 24 hours during 10 days despite of its changing morphology during cell locomotion. Each cell was tracked individually and its movement was recorded. From the data obtained, the trajectory of 8 individual cells that survived up to 10 days were drawn, and the partial or total distance covered were measured (see figure 8).

As shown in figure 9, macrophage with code 158 has travelled a total of 697 µm in 10 days. In general, cells moved more actively during the fist five days in culture. Cell locomotion indicates that cells are healthy and that the polysilicon encoded microparticles according to the present invention do not induce any cytotoxicity or damaging effects. Until now, cells were tracked using manual or automated detectors coupled to sophisticated software {{Debeir,O. 2004; Li,K. 2007}}, which makes it possible to follow the cells during one or two days. The advantage of using encoded microparticles according to the present invention is that no special equipment is needed, other than an inverted light microscope which is typically found in all cell biology laboratories.

With the aforementioned experiments, firstly it was shown that the designed microparticles can be visualized and their patterns are easily recognized under a light microscope using a 60X objective, the maximum magnification objective routinely used in cell culture laboratories. As no UV light or laser beam needs to be employed to illuminate the encoded microparticle, phototoxicity is prevented and cells are not disturbed. Furthermore, it was shown that the encoded microparticles according to the present invention can be introduced into macrophages, and that the internalized microparticles can be correctly identified using an inverted optical microscope. In addition, it was shown that polysilicon microparticles according to the present invention are biocompatible (viability of cells was not affected by the introduction of the particles). Finally, an example of the utilization of polysilicon microparticles in cell tracking has been provided, by individually following the encoded macrophages during 10 days and recording their locomotion. Furthermore

Although this has not been experimentally tested, it is expected that encoded microparticles according to the present invention may also be introduced into cell types without phagocytic capacity. It has been described that HeLa cells and fibroblasts (NIH 3T3) can endocyte particles as large as 6 µm {{Gratton,S.E.A. 2008; Javier,A.M. 2008}}. Thus, the encoded microparticles (of proper dimensions) may be usable to track a large variety of cells.

In another series of experiments, the possibilities of labeling and tracking isolated mouse embryos was investigated. In the experiments, it was investigated whether encoded microparticles according to the present invention may be introduced into the perivitelline space or attached to the zona pellucida of embryos. It was investigated whether their introduction affected the viability of embryos, whether the microparticles were retained in the embryos allowing correct identification and whether the microparticles were released from the embryos at the moment of the emergence of the blastocyst from the zona pellucida (hatching).

In the experiments, zygotes of female mice were used. Encoded microparticles of the three types A, B, and C (previously described) were in a first set of experiments introduced into the perivitelline space of the zygotes using well known microinjection techniques. The retention rate of the different types of encoded microparticles was investigated. It was found that all three types showed satisfactory retention rates during the entire in vitro culture of the embryo (retention rate > 90%).

It was further investigated whether the introduction of the encoded microparticles affected the development of the embryo. It was found that the microparticles did not significantly affect the development of the embryo. Figure 11 illustrates the development of embryos with different types of encoded microparticles. Figure 11(A1) shows one microparticle in an embryo at an early stage, comprising a single cell. Figure 11 (A2) shows the same embryo (and the same microparticle of type A) comprising two cells. Figure 11 (B3) and (B4) show an embryo comprising four cells and eight cells respectively comprising a microparticle of the B type. Figure 11 (C4) and (C5) respectively show embryos at morula and blastocyst stage comprising microparticles of the C type.

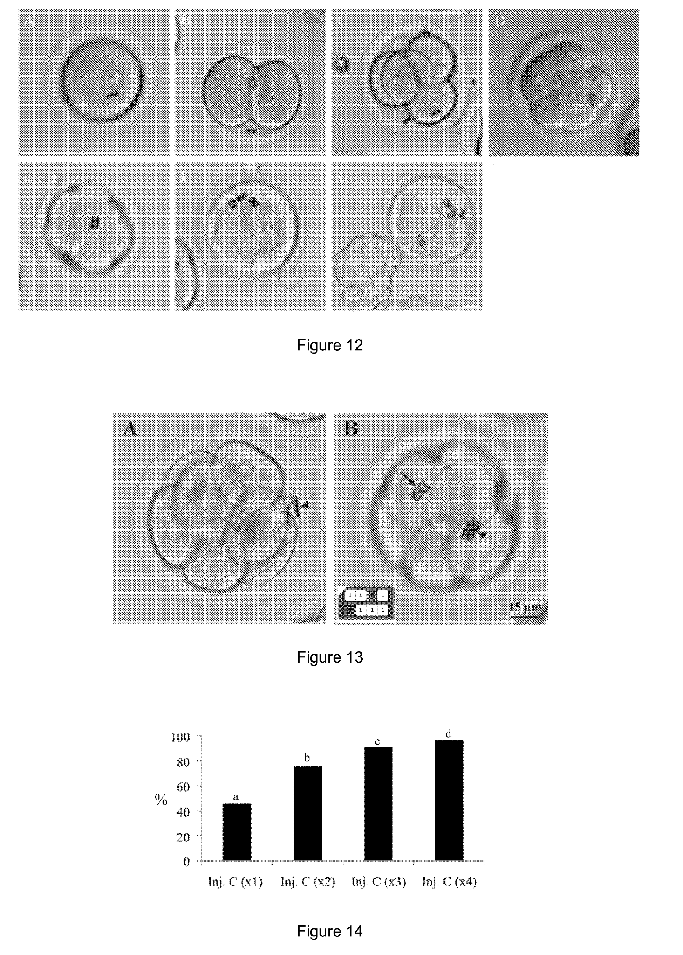

In another series of experiments, a plurality of encoded microparticles was introduced into the perivitelline space in order to increase the rate of identification. Figure 12 (A)-(G) illustrate an example. Figure 12(A) shows a zygote comprising a "B type" particle, figure 12 (B) shows an embryo comprising two cells and one A type particle. Figure 12 (C) shows an embryo comprising four cells and two A type particles. Figure 12 (D) shows an embryo comprising 8 cells with one type C particle. Figure 12 (E) shows a morula with one type C particle. Figure 12 (F) shows a blastocyst with three type C particles and figure G shows the presence of four type C microparticles in a hatching blastocyst.

Figures 13 (A) and (B) shows two different focal planes of the same embryo comprising four encoded microparticles of the type C. Both figures show an embryo comprising 8 cells and four C type microparticles in the perivitelline space. In figure 13(A), proper identification is not possible without manipulation of the embryo, whereas in figure 13 (B) clear identification is possible.

The ability to identify the embryos was found to increase with an increased number of encoded microparticles, which is illustrated in figure 14. Very importantly, it was also shown that the increased number of encoded particles (from 1 to 4) did not affect the viability of the embryos. It did affect however the retention rate of the microparticles in the perivitelline space. With the introduction of a second microparticle, the retention rate dropped to 83% which was maintained when introducing three or four microparticles.

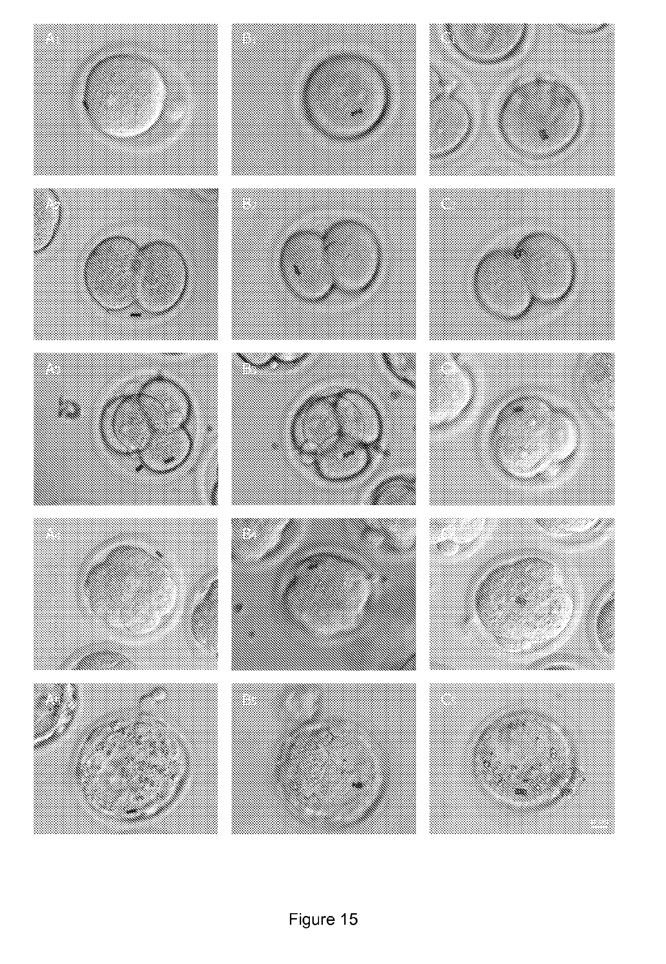

Figures 15 (A1)-(C5) show various stages of embryonic development, the embryos comprising encoded microparticles according to the present invention. The images serve to illustrate the identification possibilities of the various types of microparticles at various stages of embryonic development.

In this kind of application, it is furthermore important that at the moment of hatching, the embryo should be free from the encoded microparticles. This is important for application in assisted reproduction centres. In this way, an embryo may be tagged during its in vitro stage.

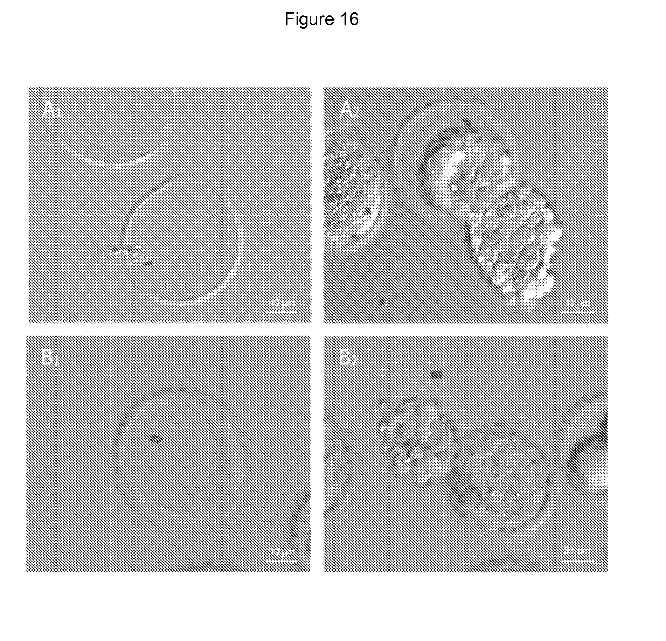

Figures 16(A1)-(B2) show the liberation of an encoded microparticle according to the present invention at the moment of hatching of the blastocyst. Figure 16 illustrates a successful liberation of the microparticle. Figures 16 (A1) and 16 (B1) show a further empty zona pellucida only comprising a microparticle. Figures 16 (A2) and 16 (B2) show liberation of the microparticle from the blastocyst at the moment of emergence of the blastocyst.

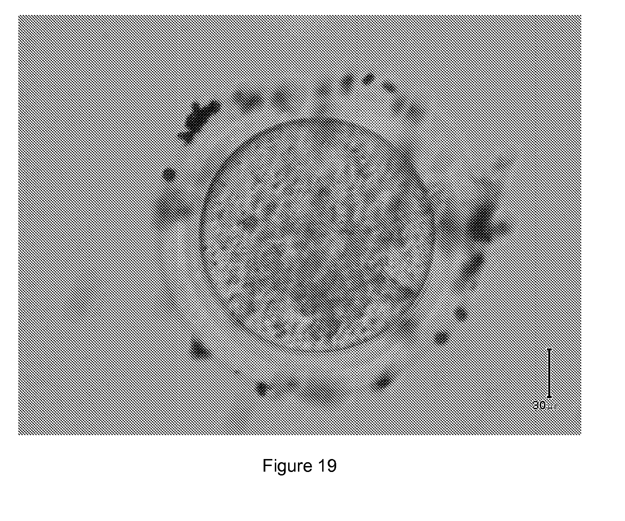

Figure 19 illustrates another way of labeling an embryo. Instead of introducing a particle in the perivitelline space, such particles are attached to the zona pellucida. Figure 19 shows an image taken with an inverted optical microscope of a mouse embryo comprising a plurality of polysilicon encoded microparticles comprising a layer of lectin attached to the zona pellucida. It has been found that polysilicon particles comprising an outer layer of lectin, preferably wheat germ agglutinin is most succesful for attaching the particle to the zona pellucida.
For the adsorption of the wheat germ agglutinin lectin (WGA; #W21405 Invitrogen) to the polysilicon microparticles, an adaptation of patent 4886761-USA (Gustafson et al., 1989) may be applied. In one method, a solution of approximately 1.05x10⁶ polysilicon microparticles in 96% ethanol was precipitated by centrifugation at 14000 rpm for 10 min. Then, supernatant was removed and the particles were dried at room temperature. Once dried, microparticles were resuspended in 20µl PBS 0.01 M with 0.1% sodium azide at pH7.4 (solution 1). This solution was sonicated for 15 min to facilitate microparticle resuspension. Then, 20 µl of solution 1 with 250 µg/ml WGA was added and incubated for 16-18 h at room temperature in agitation. Finally, after three rinses with solution 2 (PBS 0.01 M, 2.5% sucrose, 0.25% BSA, 0.05% sodium azide), the microparticles were kept at 4°C in 20 µl of PBS 0.01 M at a final concentration of 5.2x104 microparticles/ml.

For the attachment of the WGA-adsorbed microparticles to the zona pellucida of mouse zygotes, 1 µl of microparticle solution was added to a 9 µl-drop of mKSOM-H medium (Biggers et al., 2000). Then, 5 embryos were transferred into the drop, as close as possible to the microparticles. Using a mouth-controlled pipette, embryos and microparticles were pippeted together at least 3 times. Attachment of the microparticles to the zona pellucida was almost spontaneous. Embryos with the attached microparticles were washed three times in mKSOM-H and kept in culture in KSOM culture medium (EmbryoMax, Millipore) at 37°C and 5% CO2 until the blastocyst stage.

Figures 3 (a)-(f) illustrate one process by which microparticles according to the present invention may be manufactured. Although, the microparticles may be manufacture in various ways, silicon microtechnologies, used for MEMS and NEMS fabrication, are especially suitable since they allow the production of devices with dimensions in the micron range or even smaller and layers with thicknesses from several microns to few nanometers.

One way of manufacturing polysilicon encoded microparticles comprises the following steps: providing a 4" p-type (100) silicon wafers (although in other processes, other types and other dimensions of wafers may be used). A sacrificial layer is subsequently provided on top of the wafer. In this embodiment, a PECVD (Plasma Enhanced Chemical Vapour Deposition) silicon oxide layer was deposited on the front side of the wafer to be used as a sacrificial layer. In alternative processes, other CVD processes may be used. Silicon oxide is suitable to be used since it can be easily removed by HF (Hydrogen fluoride), whereas HF does not affect polysilicon. As a next step the device layer (of polysilicon), a 0.5 µm thick polysilicon was deposited using LPCVD (Low Pressure Chemical Vapour Deposition). The microparticles are subsequently given their shape by a photolithographic step and a posterior dry etching. Inductively Couple Plasma etcher (ICP, Alcatel A601) was used as it offers a 5 % etch uniformity through the whole wafer and also provides extremely vertical etching profile. Finally, the codes were released by the etching of the silicon oxide sacrificial layer after 40 minutes in vapors of acid fluoride (HF 49%). In some processes, it may be necessary to use ultrasounds in ethanol for 5 minutes to release the particles from the substrate. A scanning electron microscope image of microparticle produced in this way is shown in figure 4a.

The previously described process is especially suitable for producing type B and type C encoded microparticles. For producing type A encoded microparticles, the process needs to be adjusted a little. Patterned microparticles may be obtained using a similar process, but by vertical silicon/polysilicon dry etching using an adapted Bosch process (known to people skilled in the art). Such an adapted Bosch process may serve to form a plurality of retrusions along a cylindrical shape, by interchanging anysotropic and isotropic etching steps.

The production processes based on silicon microtechnologies allow the production of thousands of encoded microparticles with different codes, and most importantly, a mass-production with low-cost and high versatility. Silicon microtechnology allows the fabrication of robust microparticles (they do not break during production, release or cell uptake) with lateral dimensions on the micron or submicron range. Using the same microtechnology, reduction of the actual particle size to submicrometer range could be envisaged; in fact, the limiting factor is the resolution of the present optical microscopes.

For completeness, various aspects of the present invention are set out below in the following numbered clauses.
Clause 1. Encoded microparticle for labeling and/or tracking one isolated cell or isolated embryo **characterized in that** its external shape comprises a code by which it can be identified.
Clause 2. Encoded microparticle according to clause 1, further **characterized in that** its dimensions are sufficiently large that it can be viewed (and its code determined) using a normal optical microscope and/or sufficiently small that it can be introduced into or attached to a cell without causing a cell or plurality of cells to malfunction.
Clause 3. Encoded microparticle according to clause 1 or 2, further **characterized in that** it is made of a single biocompatible material.
Clause 4. Encoded microparticle according to clause 3, further **characterized in that** it is made of polysilicon or silicon.
Clause 5. Encoded microparticle according to clause 1 or 2, further **characterized in that** it is made of polysilicon or silicon covered by a layer of a lectin, preferably wheat germ agglutinin.
Clause 6. Encoded microparticle according to any preceding clause, further **characterized in that** it has an asymmetric external shape.
Clause 7. Encoded microparticle according to clause 6, further **characterized in that** its external shape comprises a distinctive marker allowing determination of the microparticle's orientation.
Clause 8. Encoded microparticle according to any previous clause, further **characterized in that** its external shape comprises a base element and a plurality of optional segments and gaps, said optional segments and gaps defining the code by which the microparticle can be identified.
Clause 9. Encoded microparticle according to clause 8, further **characterized in that** its base element is a cylinder and said plurality of optional segments is a plurality of annular protrusions around the circumference of the cylinder.
Clause 10. Encoded microparticle according to clause 8, further **characterized in that** its base element is a cylinder and said plurality of optional gaps is a plurality of annular retrusions on the circumference of the cylinder.
Clause 11. Encoded microparticle according to clause 8, further **characterized in that** its base structure is substantially flat and substantially rectangular and said plurality of optional segments is a plurality of substantially rectangular segments within said base structure.
Clause 12. Encoded microparticle according to clause 8, further **characterized in that** its base structure is substantially flat and comprises at least a substantially rectangular part, and said plurality of optional segments is a plurality of polygonal segments arranged around the perimeter of said rectangular part.
Clause 13. Encoded microparticle according to any of clauses 8-12, wherein the optional segments are of varying dimensions.
Clause 14. Encoded microparticle according to any of clauses 8-13, wherein the dimensions of the optional segments and gaps are approximately 1 µm or more.
Clause 15. Encoded microparticle according to any of clauses 11 - 14, further **characterized in that** its length and width are approximately between 2-25 µm and between 3 - 15 µm respectively.
Clause 16. Encoded microparticle according to any of clauses 11 - 14, further **characterized in that** its length and width are approximately between 6-14 µm and between 4 and 8 µm respectively.
Clause 17. Encoded microparticle according to any of clauses 11 - 14, further **characterized in that** its length and width are approximately 10 µm and 6 µm respectively.
Clause 18. Encoded microparticle according to any of clauses 11-17, further **characterized in that** its thickness is approximately between 0.3 µm - 0.8 µm, preferably approximately 0.5 µm.
Clause 19. Encoded microparticle according to clause 9 or 10, wherein the diameter and length of the cylinder are approximately between 2-4 µm and 5-15 µm respectively.
Clause 20. Encoded microparticle according to any of clauses 8 - 19, further **characterized in that** its external shape comprises a total of between 2 - 48 optional segments and gaps, preferably between 8-20 optional segments and gaps.
Clause 21. Use of one or a plurality of encoded microparticles as defined in any of the clauses 1-20 for labeling and/or tracking isolated biological material.
Clause 22. The use according to clause 21 for labeling and/or tracking an isolated cell, preferably an isolated living cell.
Clause 23. The use according to clause 22 for labeling and/or tracking a human macrophage, wherein one or a plurality of encoded microparticle is phagocyted.
Clause 24. The use according to clause 21 for labeling and/or tracking an isolated oocyte, zygote or embryo.
Clause 25. The use according to clause 24 by introducing said one or plurality of encoded microparticles in the perivitelline space.
Clause 26. The use according to clause 25, wherein a plurality of encoded microparticles comprising the same code are introduced in the perivitelline space.
Clause 27. The use according to clause 24, wherein said one or plurality of encoded microparticles are attached to the zona pellucida.
Clause 28. The use according to claim 27, wherein a plurality of encoded microparticles comprising the same code are attached to the zona pellucida.
Clause 29. A method of tracking an encoded microparticle according to any of clauses 1-20 in or attached to an isolated cell or embryo using an optical microscope.
Clause 30. A method of tracking an encoded microparticle according to clause 29 using an inverted optical microscope with an objective between 20X and 100X.
Clause 31 A method of manufacturing an encoded microparticle according to any of clauses 1-20 using silicon microtechnologies.

### BIBLIOGRAPHY

Throughout this patent application, reference was made to various articles. For the sake of completeness and clarity, a list of these scientific articles is included in the following bibliography (in alphabetical order):
Beske, O., J.J. Guo, J.R. Li, D. Bassoni, K. Bland, H. Marciniak, M. Zarowitz, V. Temov, I. Ravkin, and S. Goldbard. 2004. A novel encoded particle technology that enables simultaneous interrogation of multiple cell types. Journal of Biomolecular Screening. 9:173-185.
Biggers, J.D., L.K. McGinnis, and M. Raffin. 2000. Amino acids and preimplantation development of the mouse in protein-free potassium simplex optimized medium. Biol.Reprod. 63:281-293.
Cunin, F., T.A. Schmedake, J.R. Link, Y.Y. Li, J. Koh, S.N. Bhatia, and M.J. Sailor. 2002. Biomolecular screening with encoded porous-silicon photonic crystals. Nature Materials. 1:39-41.
Debeir, O., I. Camby, R. Kiss, P. Van Ham, and C. Decaestecker. 2004. A model-based approach for automated in vitro cell tracking and chemotaxis analyses. Cytometry Part a. 60A:29-40.
Dendukuri, D., D.C. Pregibon, J. Collins, T.A. Hatton, and P.S. Doyle. 2006. Continuous-flow lithography for high-throughput microparticle synthesis. Nature Materials. 5:365-369.
Faklaris, O., D. Garrot, V. Joshi, F. Druon, J.P. Boudou, T. Sauvage, P. Georges, P.A. Curmi, and F. Treussart. 2008. Detection of Single Photoluminescent Diamond Nanoparticles in Cells and Study of the Internalization Pathway. Small. 4:2236-2239.
Foged, C., B. Brodin, S. Frokjaer, and A. Sundblad. 2005. Particle size and surface charge affect particle uptake by human dendritic cells in an in vitro model. Int.J.Pharm. 298:315-322.
Gratton, S.E.A., P.A. Ropp, P.D. Pohlhaus, J.C. Luft, V.J. Madden, M.E. Napier, and J.M. DeSimone. 2008. The effect of particle design on cellular internalization pathways. Proc.Natl.Acad.Sci.U.S.A. 105:11613-11618.
Javier, A.M., O. Kreft, M. Semmling, S. Kempter, A.G. Skirtach, O.T. Bruns, P. del Pino, M.F. Bedard, J. Raedler, J. Kaes, C. Plank, G.B. Sukhorukov, and W.J. Parak. 2008. Uptake of Colloidal Polyelectrolyte-Coated Particles and Polyelectrolyte Multilayer Capsules by Living Cells. Adv Mater. 20:4281-4287.
Li, K., M. Chen, and T. Kanade. 2007. Cell population tracking and lineage construction with spatiotemporal context. Medical Image Computing and Computer-Assisted Intervention- MICCAI 2007, Pt 2, Proceedings. 4792:295-302.
McCarthy, J.R., F.A. Jaffer, and R. Weissleder. 2006. A macrophage-targeted theranostic nanoparticle for biomedical applications. Small. 2:983-987.
Qin, L., M.J. Banholzer, J.E. Millstone, and C.A. Mirkin. 2007. Nanodisk codes. Nano Letters. 7:3849-3853.
Tsuchiya, S., Y. Kobayashi, Y. Goto, H. Okumura, S. Nakae, T. Konno, and K. Tada. 1982. Induction of Maturation in Cultured Human Monocytic Leukemia-Cells by a Phorbol Diester. Cancer Res. 42:1530-1536.

## Claims

1. Encoded microparticle for labeling an isolated cell or an isolated embryo **characterized in that** it is made of a biocompatible material and its external shape comprises a code by which it can be identified.

2. Encoded microparticle according to claim 1, further **characterized in that** its dimensions are such that its code can be distinguished using an optical microscope and that it can be introduced into or attached to a cell without affecting the viability of a cell.

3. Encoded microparticle according to claim 1 or 2, further **characterized in that** it is made of polysilicon or silicon.

4. Encoded microparticle according to claim 3, further **characterized in that** said microparticle is made of polysilion or silicon provided with an outer layer of a lectin, preferably wheat germ agglutinin.

5. Encoded microparticle according to any previous claim, further **characterized in that** its external shape comprises a distinctive marker allowing determination of the microparticle's orientation.

6. Encoded microparticle according to any previous claim, further **characterized in that** its external shape comprises a base element and a plurality of optional segments and gaps, said optional segments and gaps defining the code by which the microparticle can be identified.

7. Encoded microparticle according to claim 6, further **characterized in that** its base element is a cylinder and said plurality of optional segments is a plurality of annular protrusions around the circumference of the cylinder.

8. Encoded microparticle according to claim 6, further **characterized in that** its base structure is substantially flat and substantially rectangular and said plurality of optional segments is a plurality of substantially rectangular segments within said base structure.

9. Encoded microparticle according to claim 6, further **characterized in that** its base structure is substantially flat and comprises at least a substantially rectangular part, and said plurality of optional segments is a plurality of polygonal segments arranged around the perimeter of said rectangular part.

10. Encoded microparticle according to any of claims 6-9, wherein the dimensions of the optional segments and gaps are approximately 1 µm or more.

11. Encoded microparticle according to any of claims 8-10, further **characterized in that** its length and width are approximately between 2-25 µm and between 3 - 15 µm respectively, preferably between 6-14 µm and between 4-8 µm respectively.

12. Encoded microparticle according to any of claims 8-11, further **characterized in that** its thickness is approximately between 0.3 µm - 0.8 µm, preferably approximately 0.5 µm.

13. Encoded microparticle according to any of claims 6-12, further **characterized in that** its external shape comprises a total of between 2 - 48 optional segments and gaps, preferably between 8-20 optional segments and gaps.

14. Use of one or a plurality of encoded microparticles as defined in any of claims 1-13 for labeling and/or tracking isolated biological material.

15. The use according to claim 14 for labeling and/or tracking an isolated cell.

16. The use according to claim 15 for labeling and/or tracking a human macrophage, wherein one or a plurality of encoded microparticle is phagocyted.

17. The use according to claim 14 for labeling and/or tracking an isolated oocyte, zygote or embryo.

18. The use according to claim 17 by introducing said one or plurality of encoded microparticles in the perivitelline space.

19. The use of according to claim 17, wherein said one or plurality of encoded microparticles are attached to the zona pellucida.

20. A method of tracking an encoded microparticle according to any of claims 1-13 introduced in or attached to an isolated cell or embryo using an optical microscope, preferably an inverted optical microscope with an objective substantially between 20X - 100X.
